# EUROPEAN PATENT APPLICATION

(11) **EP 4 772 338 A1**
(43) Date of publication of application: **08.07.2026**
(21) Application number: 23950433.5
(22) Date of filing: 30.10.2023
(51) Int. Cl.: B32B 5/26, A61F 13/15, A61F 13/49, A61F 13/496, B32B 7/14, B32B 3/08, B32B 37/12

(54) **ELASTIC LAYER FOR WEARABLE HYGIENE PRODUCT AND PREPARATION METHOD THEREFOR**

(30) Priority: 30.08.2023 CN 202311108063
(71) Applicant: SHANDONG THRIVE MATERNITY AND INFANT PRODUCTS CO., LTD, Shandong 277500 (CN)
(72) Inventor: SUN, Feng, Zaozhuang, Shandong 277500 (CN); LIANG, Jinpan, Zaozhuang, Shandong 277500 (CN); WANG, Xishan, Zaozhuang, Shandong 277500 (CN)
(74) Representative: Cabinet Beau de Loménie
(86) International application number: PCT/CN2023/127727
(87) International publication number: WO 2025/043860

(57) **Abstract**

An elastic layer for wearable hygiene products and a preparation method thereof are provided. The elastic layer for wearable hygiene products comprises: a first non-woven fabric (100), a second non-woven fabric (200), and a third non-woven fabric (300) sequentially stacked; a plurality of first elastic bands (400) provided between the first non-woven fabric (100) and the second non-woven fabric (200) with a spacing between two adjacent first elastic bands (400) being 0.2 mm to 12 mm; and a plurality of second elastic bands (500) provided between the second non-woven fabric (200) and the third non-woven fabric (300) with a spacing between two adjacent second elastic bands (500) being 0.2 mm to 12 mm, wherein the projections of the plurality of first elastic bands (400) and the plurality of second elastic bands (500) in the up-down direction are alternately arranged, so that a combined spacing between adjacent first elastic bands (400) and second elastic bands (500) is 0.1 mm to 6 mm. The elastic layer produced reduces the precision requirements for the spraying device while ensuring a small spacing by alternately arranging the projections of a plurality of first elastic bands (400) and a plurality of second elastic bands (500) in the up-down direction, thereby reducing the cost and difficulty of spraying in the production process.

## Description

### Technical Field

The present invention relates to the field of hygiene care, and particularly to an elastic layer for wearable hygiene products and a preparation method thereof.

### Background

The waistband portion of the wearable hygiene products is provided with an elastic layer to provide elasticity, thereby making diapers/pull-up pants suitable for users with different waist sizes.

The existing elastic layer generally includes non-woven fabrics on both sides and the plurality of elastic bands are added between two layers of non-woven fabrics. To ensure the elastic effect, manufacturers will choose to minimize the spacing between the elastic bands as much as possible. However, when making the elastic layer, it is usually necessary to spray glue on each elastic band. The specific operation method involves placing the plurality of elastic bands into the gaps of a dedicated spraying device with one elastic band per gap, and then spraying the elastic bands in the gaps using a spray gun. This method has the following defects:
The smaller the spacing between elastic bands, the higher the precision requirements for the gap of the spraying device. It is difficult for general spraying devices to achieve this precision requirement, which increases the cost and difficulty of spraying.

The information disclosed in this background of the present disclosure is only for enhancement of understanding of the general background of the present disclosure and may not be taken as an acknowledgement or any form of suggestion that this information forms the prior art already known to a person skilled in the art.

### Summary

The object of the present invention is to provide an elastic layer for wearable hygiene products and a preparation method thereof. The elastic layer produced reduces the precision requirements for the spraying device while ensuring a small spacing by alternately arranging the projections of a plurality of first elastic bands and a plurality of second elastic bands in the up-down direction, thereby reducing the cost and difficulty of spraying in the production process.

A first aspect of the present invention provides an elastic layer for wearable hygiene products comprising: a first non-woven fabric, a second non-woven fabric, and a third non-woven fabric sequentially stacked; a plurality of first elastic bands provided between the first non-woven fabric and the second non-woven fabric with a spacing between two adjacent elastic bands in the plurality of first elastic bands being 0.2mm to 12mm; and a plurality of second elastic bands provided between the second non-woven fabric and the third non-woven fabric with a spacing between two adjacent elastic bands in the plurality of second elastic bands being 0.2mm to 12mm, wherein the projections of the plurality of first elastic bands and the plurality of second elastic bands in the up-down direction are alternately arranged, so that a combined spacing between adjacent first elastic bands and second elastic bands is 0.1mm to 6mm.

Preferably, the plurality of first elastic bands are distributed at equal intervals, and the plurality of second elastic bands are distributed at equal intervals.

Preferably, the first elastic band has an average decitex of 10dtex-940dtex, and the second elastic band has an average decitex of 10dtex-940dtex.

Preferably, the first elastic band has a stretch range of 100%-400%, and the second elastic band has a stretch range of 100%-400%.

Preferably, the first non-woven fabric and the second non-woven fabric are bonded together through a first bonding portion, and the second non-woven fabric and the third non-woven fabric are bonded together through a second bonding portion.

A second aspect of the present invention provides a method for preparing an elastic layer for wearable hygiene products comprising: preparing a first non-woven fabric, a second non-woven fabric, and a third non-woven fabric; laying a plurality of first elastic bands on the first non-woven fabric with a spacing between two adjacent elastic bands in the plurality of the first elastic bands being 0.2mm to 12mm; laying a plurality of second elastic bands on the third non-woven fabric with a spacing between two adjacent elastic bands in the plurality of second elastic band being 0.2mm to 12mm; stacking the second non-woven fabric on the side of the first non-woven fabric where the first elastic bands are laid; and stacking the side of the third non-woven fabric laid with the second elastic bands on the second non-woven fabric, so that the projections of the plurality of first elastic bands and the plurality of second elastic bands in the up-down direction are alternately arranged, and a combined spacing between adjacent first elastic bands and second elastic bands is 0.1mm to 6mm.

Before laying the plurality of first elastic bands on the first non-woven fabric, the method further comprises spraying glue on the first elastic bands, wherein the first elastic bands can be bonded to the first non-woven fabric through the glue, and the second non-woven fabric is bonded to the first non-woven fabric through the glue sprayed on the first elastic bands.

Preferably, before laying the plurality of second elastic bands on the third non-woven fabric, the method further comprises spraying glue on the second elastic bands, wherein the second elastic band can be bonded to the third non-woven fabric through the glue, and the second non-woven fabric is bonded to the third non-woven fabric through the glue sprayed on the second elastic bands.

A third aspect of the present invention provides a method for preparing the elastic layer for wearable hygiene products comprising: preparing a first non-woven fabric, a second non-woven fabric, and a third non-woven fabric; laying a plurality of first elastic bands on a first side of the second non-woven fabric, and laying a plurality of second elastic bands on a second side of the second non-woven fabric, wherein a spacing between two adjacent elastic bands in the plurality of first elastic bands is 0.2mm to 12mm, a spacing between two adjacent elastic bands in the plurality of second elastic bands is 0.2mm to 12mm, the projections of the plurality of first elastic bands and the plurality of second elastic bands in the up-down direction are alternately arranged, and a combined spacing between adjacent first elastic bands and second elastic bands is 0.1mm to 6mm; stacking the second non-woven fabric on the first non-woven fabric; and stacking the third non-woven fabric on the second non-woven fabric.

Preferably, before laying the plurality of first elastic bands on the first side of the second non-woven fabric, the method further comprises spraying glue on the first elastic bands, wherein the first elastic bands can be bonded to the first side of the second non-woven fabric through the glue, and the second non-woven fabric is bonded to the first non-woven fabric through the glue sprayed on the first elastic bands.

Preferably, before laying the plurality of second elastic bands on the second side of the second non-woven fabric, the method further comprises spraying glue on the second elastic bands, wherein the second elastic bands can be bonded to the second side of the second non-woven fabric through the glue, and the second non-woven fabric is bonded to the third non-woven fabric through the glue sprayed on the second elastic bands.

A fourth aspect of the present invention provides a method for preparing the elastic layer for wearable hygiene products comprising: preparing a first non-woven fabric, a second non-woven fabric, and a third non-woven fabric; laying a plurality of first elastic bands on the first non-woven fabric with a spacing between two adjacent elastic bands in the plurality of the first elastic bands being 0.2mm to 12mm; stacking the second non-woven fabric on the first non-woven fabric; laying a plurality of second elastic bands on the second non-woven fabric with a spacing between two adjacent elastic bands in the plurality of second elastic bands being 0.2mm to 12mm, so that the projections of the plurality of first elastic bands and the plurality of second elastic bands in the up-down direction are alternately arranged, and a combined spacing between adjacent first elastic bands and second elastic bands is 0.1mm to 6mm; and stacking the third non-woven fabric on the second non-woven fabric.

Preferably, before laying the plurality of first elastic bands on the first non-woven fabric, the method further comprises spraying glue on the first elastic bands, wherein the first elastic bands can be bonded to the first non-woven fabric through the glue, and the second non-woven fabric is bonded to the first non-woven fabric through the glue sprayed on the first elastic bands.

Preferably, before laying the plurality of second elastic bands on the second non-woven fabric, the method further comprises spraying glue on the second elastic bands, wherein the second elastic bands can be bonded to the second non-woven fabric through the glue, and the third non-woven fabric is bonded to the second non-woven fabric through the glue sprayed on the second elastic bands.

In the elastic layer for wearable hygiene products of the present invention, the elastic layer produced reduces the precision requirements for the spraying device while ensuring a small spacing by alternately arranging the projections of a plurality of first elastic bands and a plurality of second elastic bands in the up-down direction, thereby reducing the cost and difficulty of spraying in the production process.

The methods and apparatus of the present invention have other features and advantages that will be apparent from, or will be described in detail in the accompanying drawings and subsequent embodiments incorporated herein, which together serve to explain the specific principles of the present invention.

### Brief Description of the Drawings

Figure 1 is a schematic diagram of the internal structure of an elastic layer for wearable hygiene products provided by a first embodiment of the present invention;
Figure 2 is a perspective exploded view corresponding to Figure 1;
Figure 3 is a schematic diagram of the internal structure of an elastic layer for wearable hygiene products provided by a second embodiment of the present invention;
Figure 4 is a perspective exploded view corresponding to Figure 3;
Figure 5 is a schematic diagram of the internal structure of an elastic layer for wearable hygiene products provided by a third embodiment of the present invention;
Figure 6 is a perspective exploded view corresponding to Figure 5;
Figure 7 is a schematic flowchart of a first method for preparing an elastic layer for wearable hygiene products provided by an embodiment of the present invention;
Figure 8 is a schematic flowchart of a second method for preparing an elastic layer for wearable hygiene products provided by an embodiment of the present invention;
Figure 9 is a schematic flowchart of a third method for preparing an elastic layer for wearable hygiene products provided by an embodiment of the present invention.

### Description of Reference Numerals:

| | | | |
|---|---|---|---|
| 100: | a first non-woven fabric | 200: | a second non-woven fabric |
| 300: | a third non-woven fabric | 400: | a first elastic band |
| 500: | a second elastic band. | | |

It should be understood that the accompanying drawings are not necessarily drawn to scale, but rather present a somewhat simplified representation of various features illustrative of the basic principles of the present disclosure. The specific design features disclosed by the present disclosure including, for example, specific dimensions, orientations, locations, and shapes will be determined in part by the particularly intended application and use environment.

In the figures, same reference numbers refer to the same or equivalent portions of the present disclosure throughout the several figures of the drawing.

### Embodiments

The following will refer in detail to various embodiments of the present invention, examples of which are presented in the accompanying drawings and described below. While the present disclosure will be described in conjunction with exemplary embodiments of the present disclosure, it will be understood that the present description is not intended to limit the present disclosure to those exemplary embodiments of the present disclosure. On the other hand, the present disclosure is intended to cover not only the exemplary embodiments, but also various alternatives, modifications, equivalents and other embodiments, which may be included within the spirit of the present disclosure and within the scope defined by the appended claims.

When a component is referred to as being "above" or "on" another component, the component may be in contact with the other component or there may be an intermediate component therebetween.

The wearable hygiene products mentioned in the embodiments of the present invention include but are not limited to baby diapers/pull-up pants, women's sleeping pants, and adult diapers/pull-up pants.

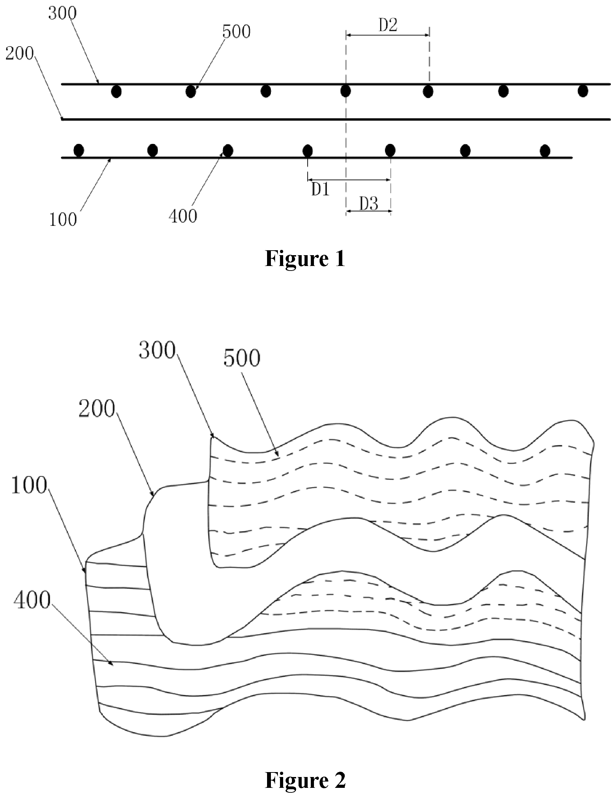

The elastic layer for wearable hygiene products according to the embodiments of the present invention will be described below with reference to Figures 1 to 9.

Figure 1 is a schematic diagram of the internal structure of an elastic layer for wearable hygiene products provided by a first embodiment of the present invention; Figure 2 is a perspective exploded view corresponding to Figure 1.

As shown in Figures 1 and 2, the elastic layer for wearable hygiene products in the embodiment of the present invention includes: a first non-woven fabric 100, a second non-woven fabric 200, a third non-woven fabric 300, a plurality of first elastic bands 400, and a plurality of second elastic bands 500 sequentially stacked.

The plurality of first elastic bands 400 are arranged between the first non-woven fabric 100 and the second non-woven fabric 200, and a spacing **D1** between two adjacent elastic bands in the plurality of first elastic bands 400 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm). Preferably, the spacing D1 between two adjacent elastic bands in the plurality of first elastic bands 400 is 2mm to 12mm.

The plurality of second elastic bands 500 are arranged between the second non-woven fabric 200 and the third non-woven fabric 300, and a spacing D2 between two adjacent elastic bands in the plurality of second elastic bands 500 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm). Preferably, the spacing D2 between two adjacent elastic bands in the plurality of second elastic bands 500 is 2mm to 12mm.

The projections of the plurality of first elastic bands 400 and the plurality of second elastic bands 500 in the up-down direction are alternately arranged, so that a combined spacing D3 between the adjacent first elastic bands 400 and second elastic bands 500 is 0.1mm to 6mm (e.g. 0.1mm, 0.2mm, 0.3mm, 0.4mm, 0.5mm, 0.6mm, 0.7mm, 0.8mm, 0.9mm, 1mm, 1.5mm, 2mm, 2.5mm, 3mm, 3.5mm, 4mm, 4.5mm, 5mm, 5.5mm, 6mm). Preferably, the combined spacing D3 between the adjacent first elastic bands 400 and second elastic bands 500 is 1mm to 6mm.

In the elastic layer for wearable hygiene products in the embodiment of the present invention, the projections of the plurality of first elastic bands 400 and the plurality of second elastic bands 500 in the up-down direction being alternately arranged allow the manufactured elastic layer to reduce the precision requirements for the spraying device while ensuring a small spacing, so as to reduce the cost and difficulty of spraying in the production process.

For example, when the spacing between adjacent elastic bands in the prior art is 3mm, the gap accuracy of the spraying device is also required to be 3mm.

When adopting the embodiments of the present invention, if the spacing between adjacent first elastic band and second elastic band is also 3mm (i.e., D3 is 3mm), due to the alternating arrangement of projections of the plurality of first elastic bands and second elastic bands in the up-down direction, the spacing between two adjacent elastic bands in the plurality of first elastic bands 400 only needs to be 6mm (i.e., D1 is 6mm), and the spacing between two adjacent elastic bands in the plurality of second elastic bands 500 only needs to be 6mm (i.e., D2 is 6mm). At this time, the gap accuracy of the spraying device only needs to be 6mm.

In the exemplary embodiment, the first non-woven fabric 100/ the second non-woven fabric 200/ the third non-woven fabric 300 can be selected from spunlace fabric, spunbond fabric, or spunmelt fabric.

When a diaper is worn on a person, the first non-woven fabric 100, the second non-woven fabric 200, the third non-woven fabric 300, the plurality of first elastic bands 400, and the plurality of second elastic bands 500 of the elastic layer for wearable hygiene products extend along the circumferential direction of the waist of the human body.

It should be noted that Figures 1 to 6 in the present invention only schematically illustrate the approximate shapes of the respective layers and the general positional relationships between layers. To better distinguish the boundaries between the layers, gaps are illustrated between the layers in Figures 1 to 6. However, in actual products, there is no gap between the first non-woven fabric 100, the second non-woven fabric 200, and the third non-woven fabric 300, but they are bonded together.

In an exemplary embodiment, the peel strength between the first non-woven fabric 100 and the second non-woven fabric 200 is about 1N/cm to about 15N/cm (e.g., 1N/cm, 2N/cm, 3N/cm, 4N/cm, 5N/cm, 6N/cm, 7N/cm, 8N/cm, 9N/cm, 10N/cm, 11N/cm, 12N/cm, 13N/cm, 14N/cm, 15N/cm).

In an exemplary embodiment, the peel strength between the second non-woven fabric 200 and the third non-woven fabric 300 is about 1N/cm to about 15N/cm (e.g., 1N/cm, 2N/cm, 3N/cm, 4N/cm, 5N/cm, 6N/cm, 7N/cm, 8N/cm, 9N/cm, 10N/cm, 11N/cm, 12N/cm, 13N/cm, 14N/cm, 15N/cm).

In the exemplary embodiment, glue is sprayed on the first elastic bands 400. The first non-woven fabric 100 is bonded to the second non-woven fabric 200 by the glue sprayed on the first elastic bands 400.

In the exemplary embodiment, glue is sprayed on the second elastic bands 500. The second non-woven fabric 200 is bonded to the third non-woven fabric 300 by the glue sprayed on the second elastic bands 500.

In addition to bonding by glue, the first elastic bands 400, the first non-woven fabric 100, and the second non-woven fabric 200, as well as the second elastic bands 500, the second non-woven fabric 200, and the third non-woven fabric 300 may also be bonded together by ultrasonic welding.

In an embodiment, as shown in Figure 1, the plurality of first elastic bands 400 are distributed at equal intervals. In another embodiment, the plurality of first elastic bands 400 can also be distributed at non-equal intervals (not illustrated in the figure).

In an embodiment, as shown in Figure 1, the plurality of second elastic bands 500 are distributed at equal intervals. In another embodiment, the plurality of second elastic bands 500 can also be distributed at non-equal intervals (not illustrated in the figure).

In an exemplary embodiment, the first elastic band 400 has an average decitex of 10dtex-940dtex (e.g. 10dtex, 50dtex, 100dtex, 150dtex, 200dtex, 250dtex, 300dtex, 350dtex, 400dtex, 440dtex, 500dtex, 600dtex, 700dtex, 800dtex, 940dtex), and the second elastic band 500 has an average decitex of 10dtex-940dtex (e.g. 10dtex, 50dtex, 100dtex, 150dtex, 200dtex, 250dtex, 300dtex, 350dtex, 400dtex, 440dtex, 500dtex, 600dtex, 700dtex, 800dtex, 940dtex).

In an exemplary embodiment, the first elastic band 400 has a stretch range of 100%-400% (e.g., 100%, 150%, 200%, 250%, 300%, 350%, 400%).

In the exemplary embodiment, the second elastic band 500 has a stretch range of 100%-400% (e.g., 100%, 150%, 200%, 250%, 300%, 350%, 400%).

In the exemplary embodiment, the elastic layer includes 10-130 (e.g. 10, 20, 30, 40, 50, 60, 70, 80, 90, 100, 110, 120, 130) first elastic bands 400.

In the exemplary embodiment, the elastic layer includes 10-130 (e.g. 10, 20, 30, 40, 50, 60, 70, 80, 90, 100, 110, 120, 130) second elastic bands 500.

In the exemplary embodiment, the first non-woven fabric 100 and the second non-woven fabric 200 are bonded together through a first bonding portion, and the second non-woven fabric 200 and the third non-woven fabric 300 are bonded together through a second bonding portion.

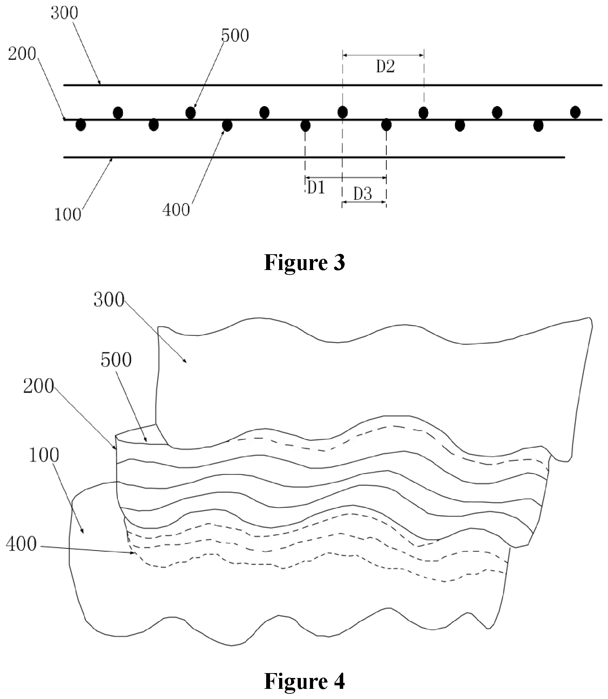

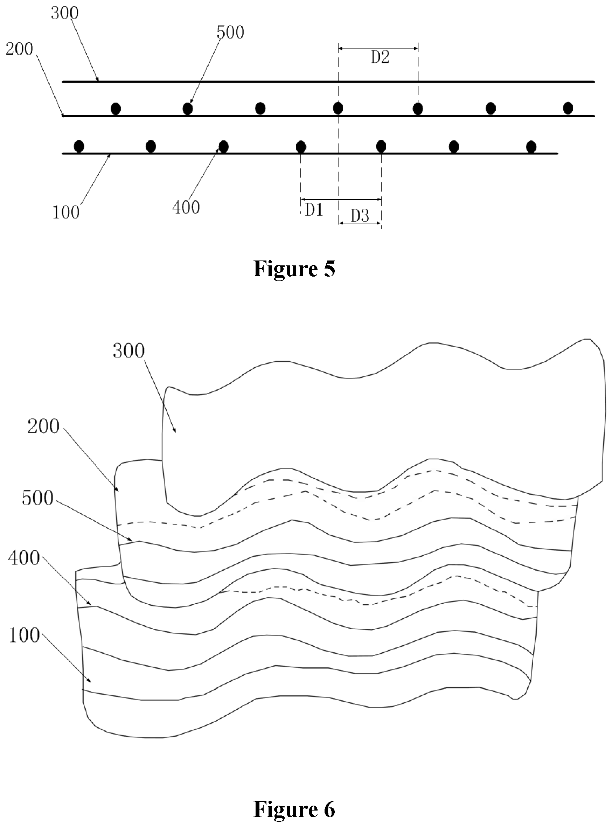

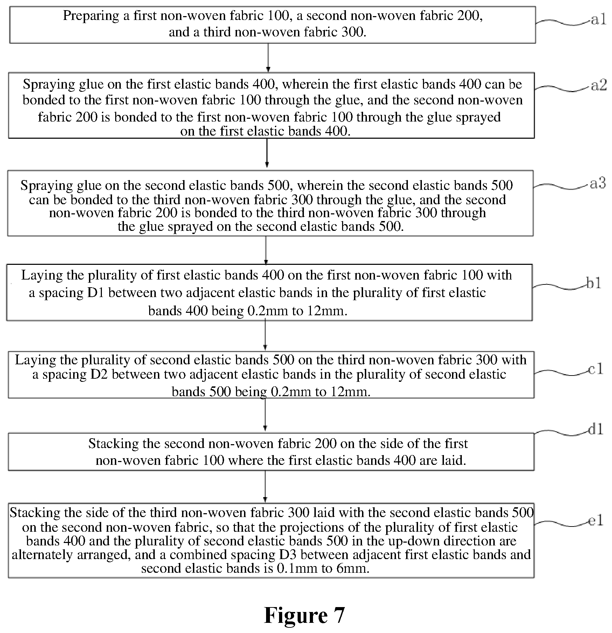

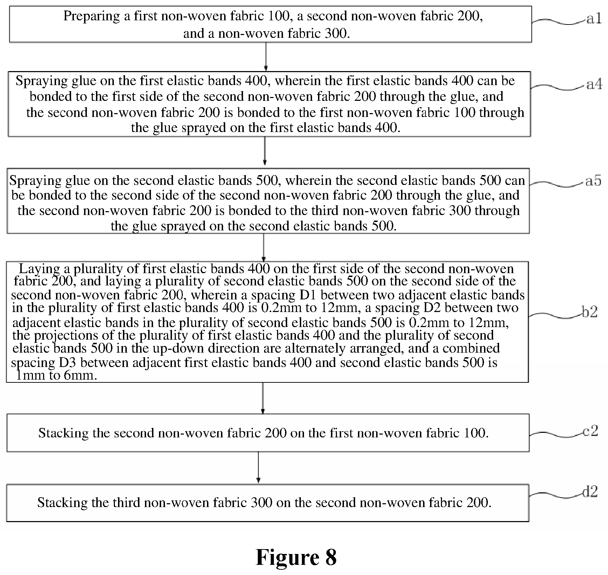

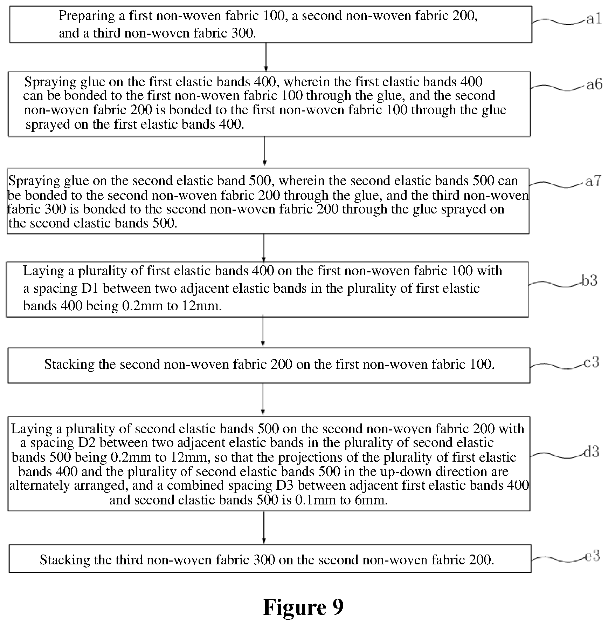

Figure 3 is a schematic diagram of the internal structure of an elastic layer for wearable hygiene products provided by a second embodiment of the present invention; Figure 4 is a perspective exploded view corresponding to Figure 3; Figure 5 is a schematic diagram of the internal structure of an elastic layer for wearable hygiene products provided by a third embodiment of the present invention; Figure 6 is a perspective exploded view corresponding to Figure 5; Figure 7 is a schematic flowchart of a first method for preparing an elastic layer for wearable hygiene products provided by an embodiment of the present invention; Figure 8 is a schematic flowchart of a second method for preparing an elastic layer for wearable hygiene products provided by an embodiment of the present invention; Figure 9 is a schematic flowchart of a third method for preparing an elastic layer for wearable hygiene products provided by an embodiment of the present invention.

The embodiments of the present invention also provide three methods for preparing an elastic layer for wearable hygiene products. The three methods are introduced below in conjunction with the accompanying drawings.

In an embodiment, as shown in Figure 7, a method of preparing an elastic layer for wearable hygiene products includes:
Step a1) preparing a first non-woven fabric 100, a second non-woven fabric 200, and a third non-woven fabric 300.
Step b1) laying a plurality of first elastic bands 400 on the first non-woven fabric 100, wherein a spacing D1 between two adjacent elastic bands in the plurality of first elastic bands 400 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm). Preferably, the spacing D1 between two adjacent elastic bands in the plurality of first elastic bands 400 is 2mm to 12mm.
Step c1) laying a plurality of second elastic bands 500 on the third non-woven fabric 300, wherein a spacing D2 between two adjacent elastic bands in the plurality of second elastic bands 500 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm). Preferably, the spacing D2 between two adjacent elastic bands in the plurality of second elastic bands 500 is 2mm to 12mm.
Step d1) stacking the second non-woven fabric 200 on the side of the first non-woven fabric 100 where the first elastic bands 400 are laid.
Step e1) stacking the side of the third non-woven fabric 300 laid with the second elastic bands 500 on the second non-woven fabric, so that the projections of the plurality of first elastic bands 400 and the plurality of second elastic bands 500 in the up-down direction are alternately arranged, and a combined spacing D3 between adjacent first elastic bands and second elastic bands is 0.1mm to 6mm (e.g. 0.1mm, 0.2mm, 0.3mm, 0.4mm, 0.5mm, 0.6mm, 0.7mm, 0.8mm, 0.9mm, 1mm, 1.5mm, 2mm, 2.5mm, 3mm, 3.5mm, 4mm, 4.5mm, 5mm, 5.5mm, 6mm). Preferably, the combined spacing D3 between adjacent first elastic bands 400 and second elastic bands 500 is 1mm to 6mm.

So far, the elastic layer for wearable hygiene products shown in Figures 1 and 2 is prepared.

In the exemplary embodiment, as shown in Figure 7, before laying the plurality of first elastic bands 400 on the first non-woven fabric 100, the above method further includes:
Step a2) spraying glue on the first elastic bands 400.

The first elastic bands 400 can be bonded to the first non-woven fabric 100 through the glue, and the second non-woven fabric 200 is bonded to the first non-woven fabric 100 through the glue sprayed on the first elastic bands 400.

In the exemplary embodiment, as shown in Figure 7, before laying the plurality of second elastic bands 400 on the third non-woven fabric 300, the above method further includes:
Step a3) spraying glue on the second elastic bands 500.

The second elastic bands 500 can be bonded to the third non-woven fabric 300 through the glue, and the second non-woven fabric 200 is bonded to the third non-woven fabric 300 through the glue sprayed on the second elastic bands 500.

In another embodiment, as shown in Figure 8, a method of preparing an elastic layer for wearable hygiene products includes:
Step a1) preparing a first non-woven fabric 100, a second non-woven fabric 200, and a third non-woven fabric 300.
Step b2) laying a plurality of first elastic bands 400 on the first side of the second non-woven fabric 200, and laying a plurality of second elastic bands 500 on the second side of the second non-woven fabric 200, wherein a spacing D1 between two adjacent elastic bands in the plurality of first elastic bands 400 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm), a spacing D2 between two adjacent elastic bands in the plurality of second elastic bands 500 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm), the projections of the plurality of first elastic bands 400 and the plurality of second elastic bands 500 in the up-down direction are alternately arranged, and a combined spacing D3 between adjacent first elastic bands 400 and second elastic bands 500 is 0.1mm to 6mm (e.g. 0.1mm, 0.2mm, 0.3mm, 0.4mm, 0.5mm, 0.6mm, 0.7mm, 0.8mm, 0.9mm, 1mm, 1.5mm, 2mm, 2.5mm, 3mm, 3.5mm, 4mm, 4.5mm, 5mm, 5.5mm, 6mm). Preferably, the spacing D1 between two adjacent elastic bands in the plurality of first elastic bands 400 is 2mm to 12mm, the spacing D2 between two adjacent elastic bands in the plurality of second elastic bands 500 is 2mm to 12mm, and the combined spacing D3 between adjacent first elastic bands 400 and second elastic bands 500 is 1mm to 6mm.
Step c2) stacking the second non-woven fabric 200 on the first non-woven fabric 100.
Step d2) stacking the third non-woven fabric 300 on the second non-woven fabric 200.

So far, the elastic layer for wearable hygiene products shown in Figures 3 and 4 is prepared.

In the exemplary embodiment, as shown in Figure 8, before laying the plurality of first elastic bands 400 on the first side of the second non-woven fabric 200, the above method further includes:
Step a4) spraying glue on the first elastic bands 400.

The first elastic bands 400 can be bonded to the first side of the second non-woven fabric 200 through the glue, and the second non-woven fabric 200 is bonded to the first non-woven fabric 100 through the glue sprayed on the first elastic bands 400.

In the exemplary embodiment, as shown in Figure 8, before laying the plurality of second elastic bands 500 on the second side of the second non-woven fabric 200, the above method further includes:
Step a5) spraying glue on the second elastic bands 500.

The second elastic bands 500 can be bonded to the second side of the second non-woven fabric 200 through the glue, and the second non-woven fabric 200 is bonded to the third non-woven fabric 300 through the glue sprayed on the second elastic bands 500.

In another embodiment, as shown in Figure 9, a method of preparing an elastic layer for wearable hygiene products includes:
Step a1) preparing a first non-woven fabric 100, a second non-woven fabric 200, and a third non-woven fabric 300.
Step b3) laying a plurality of first elastic bands 400 on the first non-woven fabric 100, wherein a spacing D1 between two adjacent elastic bands in the plurality of first elastic bands 400 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm). Preferably, the spacing D1 between two adjacent elastic bands in the plurality of first elastic bands 400 is 2mm to 12mm.
Step c3) stacking the second non-woven fabric 200 on the first non-woven fabric 100.
Step d3) laying a plurality of second elastic bands 500 on the second non-woven fabric 200, wherein a spacing D2 between two adjacent elastic bands in the plurality of second elastic bands 500 is 0.2mm to 12mm (e.g. 0.2mm, 0.3mm, 0.4mm, 0.5mm, 0.6mm, 0.7mm, 0.8mm, 0.9mm, 1mm, 1.1mm, 1.2mm, 1.3mm, 1.4mm, 1.5mm, 1.6mm, 1.7mm, 1.8mm, 1.9mm, 2mm, 3mm, 4mm, 5mm, 6mm, 7mm, 8mm, 9mm, 10mm, 11mm, 12mm), such that the projections of the plurality of first elastic bands 400 and the plurality of second elastic bands 500 in the up-down direction are alternately arranged, and a combined spacing D3 between adjacent first elastic bands 400 and second elastic bands 500 is 0.1 mm to 6mm (e.g. 0.1mm, 0.2mm, 0.3mm, 0.4mm, 0.5mm, 0.6mm, 0.7mm, 0.8mm, 0.9mm, 1mm, 1.5mm, 2mm, 2.5mm, 3mm, 3.5mm, 4mm, 4.5mm, 5mm, 5.5mm, 6mm). Preferably, the spacing D2 between two adjacent elastic bands in the plurality of second elastic bands 500 is 2mm to 12mm, and the combined spacing D3 between adjacent first elastic bands 400 and second elastic bands 500 is 1mm to 6mm.
Step e3) stacking a third non-woven fabric 300 on the second non-woven fabric 200.

So far, the elastic layer for wearable hygiene products shown in Figures 5 and 6 is prepared.

In the exemplary embodiment, as shown in Figure 9, before laying the plurality of first elastic bands 400 on the first non-woven fabric 100, the above method further includes:
Step a6) spraying glue on the first elastic bands 400.

The first elastic bands 400 can be bonded to the first non-woven fabric 100 through the glue, and the second non-woven fabric 200 is bonded to the first non-woven fabric 100 through the glue sprayed on the first elastic bands 400.

In the exemplary embodiment, as shown in Figure 9, before laying the plurality of second elastic bands 500 on the second non-woven fabric 200, the above method further includes:
Step a7) spraying glue on the second elastic bands 500.

The second elastic bands 500 can be bonded to the second non-woven fabric 200 through the glue, and the third non-woven fabric 300 is bonded to the second non-woven fabric 200 through the glue sprayed on the second elastic bands 500.

The embodiments of the present invention further provide wearable hygiene products comprising the elastic layer for wearable hygiene products as described above.

For convenience in explanation and accurate definition in the appended claims, the terms "up", "down", "inner", "outer", "above", "below", "upper", "lower", "upward", "downward", "front", "rear", "back", "inside", "outside", "inward", "outward", "interior", "exterior", "internal", "external", "forward", and "backward" are used to describe features of the exemplary embodiments with reference to the positions of such features as shown in the figures.

The foregoing descriptions of specific exemplary embodiments of the present disclosure have been presented for purposes of illustration and description. The foregoing description is not intended to be exhaustive, nor is it intended to limit the present disclosure to the precise forms disclosed, and it is apparent that many changes and variations are possible in light of the above teachings. The exemplary embodiments are selected and described in order to explain certain principles of the invention and its practical application, so that the skilled in the art can realize and utilize various exemplary embodiments of the present disclosure, as well as various alternatives and modifications thereof. The scope of the present invention is defined by the appended claims and their equivalents.

## Claims

1. An elastic layer for wearable hygiene products comprising:
a first non-woven fabric, a second non-woven fabric, and a third non-woven fabric sequentially stacked;
a plurality of first elastic bands provided between the first non-woven fabric and the second non-woven fabric with a spacing between two adjacent elastic bands in the plurality of first elastic bands being 0.2mm to 12mm; and
a plurality of second elastic bands provided between the second non-woven fabric and the third non-woven fabric with a spacing between two adjacent elastic bands in the plurality of second elastic bands being 0.2mm to 12mm;
wherein the projections of the plurality of first elastic bands and the plurality of second elastic bands in the up-down direction are alternately arranged, so that a combined spacing between adjacent first elastic bands and second elastic bands is 0.1mm to 6mm.

2. The elastic layer for wearable hygiene products according to claim 1, wherein the plurality of first elastic bands are distributed at equal intervals, and the plurality of second elastic bands are distributed at equal intervals.

3. The elastic layer for wearable hygiene products according to claim 2, wherein the first elastic band has an average decitex of 10dtex-940dtex, and the second elastic band has an average decitex of 10dtex-940dtex.

4. The elastic layer for wearable hygiene products according to claim 3, wherein the first elastic band has a stretch range of 100%-400%, and the second elastic band has a stretch range of 100%-400%.

5. The elastic layer for wearable hygiene products according to claim 1, wherein the first non-woven fabric and the second non-woven fabric are bonded together through a first bonding portion, and the second non-woven fabric and the third non-woven fabric are bonded together through a second bonding portion.

6. A method for preparing an elastic layer for wearable hygiene products comprising:
preparing a first non-woven fabric, a second non-woven fabric, and a third non-woven fabric;
laying a plurality of first elastic bands on the first non-woven fabric with a spacing between two adjacent elastic bands in the plurality of the first elastic bands being 0.2mm to 12mm;
laying a plurality of second elastic bands on the third non-woven fabric with a spacing between two adjacent elastic bands in the plurality of second elastic band being 0.2mm to 12mm;
stacking the second non-woven fabric on the side of the first non-woven fabric where the first elastic bands are laid; and
stacking the side of the third non-woven fabric laid with the second elastic bands on the second non-woven fabric, so that the projections of the plurality of first elastic bands and the plurality of second elastic bands in the up-down direction are alternately arranged, and a combined spacing between adjacent first elastic bands and second elastic bands is 0.1mm to 6mm.

7. The method for preparing the elastic layer for wearable hygiene products according to claim 6, wherein before laying the plurality of first elastic bands on the first non-woven fabric, the method further comprises:
spraying glue on the first elastic bands,
wherein the first elastic bands can be bonded to the first non-woven fabric through the glue, and the second non-woven fabric is bonded to the first non-woven fabric through the glue sprayed on the first elastic bands.

8. The method for preparing the elastic layer for wearable hygiene products according to claim 6, wherein before laying the plurality of second elastic bands on the third non-woven fabric, the method further comprises:
spraying glue on the second elastic bands,
wherein the second elastic band can be bonded to the third non-woven fabric through the glue, and the second non-woven fabric is bonded to the third non-woven fabric through the glue sprayed on the second elastic bands.

9. A method for preparing the elastic layer for wearable hygiene products comprising:
preparing a first non-woven fabric, a second non-woven fabric, and a third non-woven fabric;
laying a plurality of first elastic bands on a first side of the second non-woven fabric, and laying a plurality of second elastic bands on a second side of the second non-woven fabric, wherein a spacing between two adjacent elastic bands in the plurality of first elastic bands is 0.2mm to 12mm, a spacing between two adjacent elastic bands in the plurality of second elastic bands is 0.2mm to 12mm, the projections of the plurality of first elastic bands and the plurality of second elastic bands in the up-down direction are alternately arranged, and a combined spacing between adjacent first elastic bands and second elastic bands is 0.1 mm to 6mm;
stacking the second non-woven fabric on the first non-woven fabric; and
stacking the third non-woven fabric on the second non-woven fabric.

10. The method for preparing the elastic layer for wearable hygiene products according to claim 9, wherein before laying the plurality of first elastic bands on the first side of the second non-woven fabric, the method further comprises:
spraying glue on the first elastic bands,
wherein the first elastic bands can be bonded to the first side of the second non-woven fabric through the glue, and the second non-woven fabric is bonded to the first non-woven fabric through the glue sprayed on the first elastic bands.

11. The method for preparing the elastic layer for wearable hygiene products according to claim 9, wherein before laying the plurality of second elastic bands on the second side of the second non-woven fabric, the method further comprises:
spraying glue on the second elastic bands,
wherein the second elastic bands can be bonded to the second side of the second non-woven fabric through the glue, and the second non-woven fabric is bonded to the third non-woven fabric through the glue sprayed on the second elastic bands.

12. A method for preparing the elastic layer for wearable hygiene products comprising:
preparing a first non-woven fabric, a second non-woven fabric, and a third non-woven fabric;
laying a plurality of first elastic bands on the first non-woven fabric with a spacing between two adjacent elastic bands in the plurality of the first elastic bands being 0.2mm to 12mm;
stacking the second non-woven fabric on the first non-woven fabric;
laying a plurality of second elastic bands on the second non-woven fabric with a spacing between two adjacent elastic bands in the plurality of second elastic bands being 0.2mm to 12mm, so that the projections of the plurality of first elastic bands and the plurality of second elastic bands in the up-down direction are alternately arranged, and a combined spacing between adjacent first elastic bands and second elastic bands is 0.1mm to 6mm; and
stacking the third non-woven fabric on the second non-woven fabric.

13. The method for preparing the elastic layer for wearable hygiene products according to claim 12, wherein before laying the plurality of first elastic bands on the first non-woven fabric, the method further comprises:
spraying glue on the first elastic bands,
wherein the first elastic bands can be bonded to the first non-woven fabric through the glue, and the second non-woven fabric is bonded to the first non-woven fabric through the glue sprayed on the first elastic bands.

14. The method for preparing an elastic layer for wearable hygiene products according to claim 12, wherein before laying the plurality of second elastic bands on the second non-woven fabric, the method further comprises:
spraying glue on the second elastic bands,
wherein the second elastic bands can be bonded to the second non-woven fabric through the glue, and the third non-woven fabric is bonded to the second non-woven fabric through the glue sprayed on the second elastic bands.
